Europäisches Patentamt

**European Patent Office**

**Office européen des brevets**

(19)

(11) Publication number: **0 186 523**
**A2**

## (12) EUROPEAN PATENT APPLICATION

(21) Application number: **85309522.2**

(22) Date of filing: **30.12.85**

(51) Int. Cl.⁴: **C 12 N 11/04**
**C 12 N 11/06, C 12 N 11/08**

(30) Priority: **28.12.84 US 687209**

(43) Date of publication of application:
**02.07.86 Bulletin 86/27**

(84) Designated Contracting States:
**AT BE CH DE FR GB IT LI LU NL SE**

(71) Applicant: **GENEX CORPORATION**
**16020, Industrial Drive**
**Gaithersburg Maryland 20877(US)**

(72) Inventor: **Swann, Wayne Elliott**
**5392, Storm Drift**
**Columbia Maryland 21045(US)**

(74) Representative: **Holmes, Michael John et al,**
**Frank B. Dehn & Co. European Patent Attorneys Imperial**
**House 15-19 Kingsway**
**London, WC2B 6UZ,(GB)**

(54) **Insolubilized biological material composites.**

(57) The present invention provides an insolubilized biologic-
al material composite comprising a biologically active material immobilized within a copolymer which is the reaction
product of (a) an epoxy resin having two or more epoxide
groups per molecule and (b) a polyalkyleneimine.

- 1 -

## INSOLUBILIZED BIOLOGICAL MATERIAL COMPOSITES

The present invention relates to insolubilized biological material composites. In particular, it relates to the immobilization of biological materials within epoxy resin/polyalkyleneimine copolymers.

Biological materials, such as enzymes, enzyme producing microorganisms and cells, are often used as catalysts for synthetic reactions and for analytical techniques. Such catalysts are desirable because they operate with high specificity and efficiency under generally mild reaction conditions.

Because enzymes and other biocatalysts are generally water-soluble, they are suited for use in batch-type reaction systems. Reuse of such enzymes and other biocatalysts is limited because of difficulties in recovering those materials from the spent reaction medium in active or usable form. Moreover, the materials tend to remain in the prepared product as contaminants. In order to avoid these problems, methods have been developed to immobilize biological materials which exhibit catalytic activity on insoluble solid supports. Immobilization is intended to result in a stabilized biological material which can withstand the rigors of repeated or continuous use.

Several immobilization systems for biological materials have been reported. Enzymes have been immobilized by absorption onto insoluble materials such as charcoal, glass, cellulose, calcium phosphate gel, montmorillonite and organic ion-exchange resins among others. Immobilization has also been achieved by entrapment within starch and acrylamide gels, covalent attachment between enzymes and insoluble organic polymers, as well as covalent attachment between enzyme molecules themselves.

The processes of the prior art often result in products of reduced enzymatic activity, when compared with that of the corresponding unbound biological material. These biological materials are known to be sensitive to thermal and chemical denaturation or inactivation. The loss of biological activity often results when immobilizing operations are carried out under harsh conditions which can be particularly problematic when polymer condensation reactions are involved. Furthermore, the products resulting from prior art methods are often disadvantageous with respect to their hydrophilicity, strength, durability, and porosity.

Attempts also have been made to immobilize certain biological materials using compounds containing epoxy linkages. These efforts generally have not produced favorable results for although the epoxy functionality is very reactive towards amine and hydroxyl groups in the biological materials, immobiliztion systems based on epoxy moieties heretofore have been considered too hydrophobic for application as biocatalysts.

It recently has been discovered that in many instances biological materials can be immobilized such that they retain a high degree of their catalytic acitivity by entrapping them within a condensed polyalkyleneimine polymer. In accordance with the method detailed in U.S. Patent 4,434,228, a polyalkyleneimine polymer is condensed with a polycarboxylic acid in the presence of a condensing agent. The biological material to be immobilized is mixed with the polyalkyleneimine polymer during the condensation reaction.

Although this method of immobilizing biological materials has proved advantageous in many situations, further improvements are sought. An object of this invention is to develop a method for immobilizing biological materials wherein a high degree of the

0186523

- 3 -

material's catalytic activity is retained. It also is an object of this invention to develop such a method wherein the hydrophilic-hydrophobic nature of the final product can easily be varied.

According to one aspect of the present invention, we provide an insolubilized biological material composite comprising a biologically active material immobilized within a copolymer which is the reaction product of (a) an epoxy resin having two or more epoxide groups per molecule and (b) a polyalkyleneimine. The copolymer also may comprise an effective amount of one or more cross-linkers. By varying the molar ratio of polyalkyleneimine and epoxy, one can achieve a variety of copolymers having a range of hydrophobic/hydrophilic properties. The copolymer composites of the present invention exhibit excellent strength and durability and are suitable for immobilizing a wide range of biological materials.

According to a further aspect of the present invention, we provide a method for the immobilization of a biological material which comprises the step of incorporating said biological material within a copolymer which is the reaction product of (a) an epoxy resin having two or more epoxide groups and (b) a polyalkyleneimine.

In accordance with the present invention, various biological materials, including cells and enzymes, can be immobilized by contacting them with a copolymer which is the reaction product of an epoxy resin having two or more epoxide groups per molecule, a polyalkyleneimine and, optionally, a suitable cross-linking agent. Examples of biological materials which can be immobilized include free enzymes, whole or ruptured microbial cells, antigens, antibodies, antibiotics, carbohydrates, coenzymes and plant and animal cells. Enzymes can be added to the copolymer in aqueous solution or powdered form, preferably the latter. Cells can be added in the form of a wet paste or as particulates.

Preferably, the epoxy resin has a number average molecular weight of between about 150 and 2000.

The epoxy resin can be a liquid or solid and can be either a single epoxy resin or a mixture of suitable epoxy resins.

Examples of suitable diepoxides include, but are not limited to: condensation products of bisphenol-A with epichlorohydrin, examples of which are commercially available as Epon 826, 828, 1001 and 1002 (marketed by Shell Chemical Company); Araldite 6010 and 6020 (marketed by Ciba-Giegy Corp.); ester type diepoxides such as diglycidyl phthalate, diglycidyl adipate, and diglycidyl glutarate; cycloaliphatic diepoxides such as vinyl cyclohexane diepoxide and Araldite CY-179 (marketed by Ciba Geigy Corp.); and aliphatic ether type diepoxides such as ethylene glycol diglycidyl ether, 1,2-propylene glycol diglycidyl ether and 1,4 butane diol diglycidyl ether (Araldite RD-2 marketed by Ciba-Geigy Corp.).

The copolymers of the present invention are formed by contacting the above epoxy compounds with a polyalkyleneimine. Polyalkyleneimines are polymers which can be synthesized by the acid-catalyzed addition polymerization of alkyleneimine monomers. Such polymers generally vary between 30,000 and 100,000 in molecular weight, depending upon reaction conditions, and preferably have a branched chain structure.

Polyethyleneimine (PEI) is a preferred polyalkyleneimine because it currently is readily available at relatively low cost, and it functions well in the reactions employed in the present method. Polyethyleneimine is produced by ring-opening polymerization of ethyleneimine in the presence of catalysts, such as mineral acids. The polymer is highly branched and contains primary, secondary and tertiary amino groups. PEI is water soluble and when mixed with an epoxy resin in accordance with the method of this invention, a water-insoluble product results.

The molar ratio of polyalkyleneimine to the epoxy resin can vary widely. The molar ratio of the epoxy resin to polyalkyleneimine generally ranges from about 99.1 to 1.99. A more preferred ratio is from about 75:25 to about 25:75. In forming the novel copolymers of the present invention, the higher the ratio of epoxy resin to polyalkyleneimine the higher the hydrophobicity of the resultant copolymer. Alternatively, a copolymer having a higher ratio of polyalkyleneimine to epoxy is more hydrophilic.

By varying the amount of cross-linking in the copolymer of the present invention one can directly control the hydrophilicity/hydrophobicity of the copolymer. The amount of cross-linking can be controlled not only by varying the nature of the epoxy and the ratio of epoxy to polyalkyleneimine but also through the addition of one or more cross-linking agents. For example, multifunctional amines can be added to further cross-link the epoxy polymeric units of the copolymer. The amount of epoxy cross-linker employed varies in accordance with the number of epoxy functionalities of the particular epoxy resin chosen. Generally the amount of the epoxy cross-linker ranges from about 0.001 mole to about 50 moles per mole of epoxy used. Preferably from about 0.01 moles to about 10 moles are used. Suitable cross-linkers include tetraethylenepentamine (TEPA) and ethylenediamine.

In specific embodiments of the present invention, the copolymer additionally can be treated with a cross-linking agent which will provide it with additional stability and strength. Suitable cross-linkers include glutaric dialdehyde, diisocyanates, polyisocyanates, 2,4,6-trichloro-S-triazine, bisoxirane, bisimidate, divinylsulfone, 1,5-difluoro-2,4-dinitrobenzene.

Glutaric dialdehyde is the preferred amine cross-linker.

In preparing the composites, any order of addition of ingredients may be employed. For example, the biological material, polyalkyleneimine, epoxy resin and any additional cross-linker may simply be mixed together to form the final copolymer biological material composite. Alternatively, the polyalkyleneimine and a cross-linker for the epoxy groups can be mixed together, then added to the epoxy resin and biological material. Other embodiments are readily apparent to one skilled in the art.

Immobilization of biological materials within the copolymer can occur by physical entrapment as well as by covalent bonding between the polyalkyleneimine or epoxy resin and reactive groups on the biological material. For example, amine groups of the biological material can substitute for an amine group on the polyalkyleneimine and ultimately become covalently linked to the polymer.

The copolymer-biological material composite can be made by mixing the components together at room temperature. Alternatively, higher temperatures can be employed to increase the rate of polymerization. The amount of heat that can be added is dependent upon the nature and sensitivity of the particular biological material to be immobilized. The temperature chosen should be one which will not inactivate the biological material.

The copolymer can be formed in the presence of a hardener. Hardeners, such as certain polyfunctional amines, increase the reaction rate of the formation of the copolymer at room temperature. One compound useful as a hardener is HY 956 produced by the Ciba Giegy Corp.

This compound is a triethylenetetramine which is modified.

The amount of biological material added to the copolymer can vary according to the specific end use of the biological material composite. Generally, the amount ranges from about 0.001 to about 10 grams (dry weight basis) per gram of polyalkyleneimine-epoxy copolymer used, although both larger and smaller amounts may be used in accordance with the particular purpose desired for the final composite.

In one embodiment of this invention, the biological material, such as whole wet cells harvested from a fermentation broth, can be contacted with vermiculite particles. The biological material is absorbed into the vermiculite, then the vermiculite is mixed with, and coated by, the epoxy-polyalkyleneimine copolymer of this invention to form the final composite. The vermiculite acts as a carrier for the biological materials. Vermiculite particles can absorb large quantities of the materials, resulting in high density loading. The particle size of the vermiculite can vary from a fine powder to about 1 cm, preferably about 0.5 to 1 mm.

A copolymer-biological material composite of the present invention can be made in a variety of formed products such as beads, test tubes, cylinders, films and particles. The copolymers also can be used as coatings on a variety of preformed items and solid supports such as glass fibers, charcoal, cellulose, calcium phosphate gel, montmorillonite, ion exchange resins and rods. The composite can be used in batch reactions, for it can easily be separated from reaction mixtures by simple filtration, or it can be used in continuous reaction processes such as those wherein a reacting substrate flows through a packed column reactor.

By the use of the described methods and product of the present invention, it is possible to immobilize a wide variety of biological materials. The following examples describe the manner and process of making and using the invention and set forth various embodiments of the invention, but are not to be construed as limiting.

## EXAMPLE I

Ten grams of Araldite 6010 (a liquid epoxy resin commercially available from Ciba Geigy Corp.) and 10 grams of polyethyleneimine (PEI) were mixed at room temperature without a solvent. The resulting material was flexible and hydrophilic. The cure time was about 2 hours.

## EXAMPLE II

The procedure of Example I was repeated except 3 grams of TEPA was added to accelerate the speed of curing. The resulting polymer was formed within minutes and was set after 30 minutes.

## EXAMPLE III

Five grams of Araldite 6010, 10 grams of PEI and 2 grams of TEPA were mixed together. The resulting polymer was formed in a cure time of about 15 minutes.

## EXAMPLE IV

Five grams of PEI, 10 grams of Araldite 507 (an epoxy resin commercially available from Ciba Geigy Corp.) and 2.5 grams of HY 956 (polyamine hardener commercially available from Ciba Geigy Corp.) were mixed. The resulting polymer was hard and hydrophobic.

### EXAMPLE V

2.5 grams of the liquid epoxy resin Araldite 6010 and 2.5 grams of a phenylalanine ammonia lyase (PAL) producing strain of Rhodotorula rubra whole yeast cells were added to a reaction vessel and mixed. 0.5 grams of polyethleneimine (PEI) then were added and mixed. After about two hours the copolymer was cured. The copolymer was ground in a mortar with pestle, then washed with water and about 200 ml of a substrate solution comprising 267.6mM t-cinnamic acid and 8M ammonia and having a pH of 10.5. The copolymer retained a red coloration due to the color of the cells; however, the cells were not detected in the wash solutions.

### EXAMPLE VI

The general procedure of Example V was followed except 6 grams of cell paste, 10 grams of Araldite 6010, 2.5 grams of PEI and 0.5 grams of tetraethylene pentamine (TEPA) were used. The material was left to sit overnight before grinding and washing. The material was a semi-hard flexible copolymer with red coloration which retained cell mass. The material was used to convert ammonium cinnamate to L-phenylalanine by adding to it the substrate solution described in Example V. The immobilized yeast cells containing the phenylalanine enzyme were successful in making L-phenylalanine from cinnamic acid and ammonia.

### EXAMPLE VII

Five grams of Araldite 6010 and 3 grams of liquid glucoamylase enzyme preparation (commercially available from Enzyme Development Corporation) were added to a reaction vessel and mixed. Ten grams of PEI then were added and mixed. The resulting material was tested for enzymatic activity using a solution of 10-DE maltodextrin as the substrate (pH of 4.5 and 30% solids). One gram of the particles was added to 25cc of the substrate (60°C). Seventy-five percent of the starch were converted to glucose. The particles then were removed from the substrate, washed, then added to 25cc of fresh substrate solution. In the second run, 79% of the starch was found to be converted to glucose.

## EXAMPLE VIII

The general procedure for making the biological material-containing copolymer of Example VII can be followed, but before the copolymer is cured, a small amount of the liquid solution is pulled through a piece of Tygon® tubing having an inside diameter of approximately 2mm. Enough of the solution is used to coat the inner surface of the tubing. The coated tube then is placed under a heat lamp at 45°C for about 30 minutes to cure the coating solution.

The coated tube then can be used as a continuous flow reactor to convert 10-DE maltodextrin to glucose as in Example VII.

The same procedure can be used with a variety of biological materials, epoxides, and polyalkylene-imides.

## EXAMPLE IX

The general procedure for making the biological material-containing copolymer of Example VII can be followed, but before the material is fully cured, the liquid prepolymer solution is used to coat the bottom half of a glass test tube. The coated tube then is coated with a second PEI-epoxy prepolymer solution and cured. This last step is repeated a sufficient number of times to build up the thickness of the polymer coating

11

until it is rigid and self-supporting. After the final curing step, the coated tube is soaked in an aqueous solution at 35°C. The polymer expands and slips off the glass test tube to provide a self-sustaining polymeric test tube, the inside surface of which is coated with glucoamylase. This tube then can be used as a reactor to convert 10-DE maltodextrin to glucose as in Example VII.

## EXAMPLE X

One gram of PEI was mixed with 0.5 grams of 1,4 butane diol diglycidyl ether. The material was left to sit for 16 hours. The resulting cured polymeric material was hydrophillic in nature and suitable for use as a biological support material.

## Claims

1.    An insolubilized biological material composite comprising a biologically active material immobilized within a copolymer which is the reaction product of (a) an epoxy resin having two or more epoxide groups per molecule and (b) a polyalkyleneimine.

2.    A composite as claimed in claim 1 wherein said polyalkyleneimine is polyethyleneimine.

3.    A composite as claimed in claim 1 or 2 wherein the molar ratio of the epoxy resin to polyalkyleneimine is from about 99:1 to 1:99.

4.    A composite as claimed in claim 3 wherein the molar ratio of the epoxy resin to polyalkyleneimine is from about 75:25 to about 25:75.

5.    A composite as claimed in any one of claims 1 to 4 wherein said polyalkylenemine has a molecular weight in the range 30,000 to 100,000.

6.    A composite as claimed in any one of claims 1 to 5 wherein said epoxy resin has a number average molecular weight of between about 150 and 2000.

7.    A composite as claimed in any one of claims 1 to 6 wherein said epoxy resin is a condensation product of bisphenol-A with epichlorohydrin.

8.    A composite as claimed in any one of claims 1 to 7 wherein said epoxy resin is an ester-type diepoxide.

9.    A composite as claimed in any one of claims 1 to 7 wherein wherein said epoxy resin is a cycloaliphatic diepoxide.

10. A composite as claimed in any one of claims
1 to 7 wherein said epoxy resin is an aliphatic ether
diepoxide.

11. A composite as claimed in any one of claims
1 to 10 wherein the co-polymer also comprises one
or more cross-linking agents.

12. A composite as claimed in claim 11 wherein
the co-polymer comprises a cross-linking agent selected
from multifunctional amines, glutaric dialdehyde,
diisocyanates, polyisocyanates, 2,4,6-trichloro-5-
triazine, bisoxirane, bisimidate, divinylsulfone
and 1,5-difluoro-2,4-dinitrobenzene.

13. A composite as claimed in any one of claim
1 to 12 wherein the biological material is absorbed
within vermiculite.

14. A composite as claimed in any one of claims
1 to 13 wherein the immobilized biological material
is a free enzyme.

15. A composite as claimed in any one of claims
1 to 13 wherein the immobilized bilogical material
comprises whole or ruptured microbial cells.

16. A composite as claimed in any one of claims
1 to 15 wherein said composite is coated on a preformed article or solid support.

17. A composite as claimed in any one of claims
1 to 15 wherein said composite is in the form of
beads, test tubes, cylinders, films or particles.

18. A method for the preparation of a composite
as claimed in claim 1 which comprise the step of
incorporating said biological material within said
copolymer.